(19) Europäisches Patentamt
European Patent Office
Office européen des brevets

(11) EP 1 254 134 B1

(12) **FASCICULE DE BREVET EUROPEEN**

(45) Date de publication et mention de la délivrance du brevet:
**23.07.2003 Bulletin 2003/30**

(21) Numéro de dépôt: **01907687.6**

(22) Date de dépôt: **24.01.2001**

(51) Int Cl.[7]: **C07D 413/04**, A61K 31/404, A61P 43/00

(86) Numéro de dépôt international:
**PCT/FR01/00228**

(87) Numéro de publication internationale:
**WO 01/055134 (02.08.2001 Gazette 2001/31)**

# (54) DERIVES DE 1,3-DIHYDRO-2H-INDOL-2-ONE ET LEUR UTILISATION EN TANT QUE LIGANDS POUR LES RECEPTEURS V1B OU V1B ET V1A DE L'ARGININE-VASOPRESSINE

1,3-DIHYDRO-2H-INDOL-2-ON DERIVATE UND DEREN VERWENDUNG ALS LIGANDEN DER ARGININ-VASOPRESSIN REZEPTOREN V1B ODER V1B UND V1A

1,3-DIHYDRO-2H-INDOL-2-ONE DERIVATIVES,AND THEIR USE AS LIGANDS FOR V1B OR V1B AND V1A ARGININE-VASOPRESSIN-RECEPTORS

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **25.01.2000 FR 0000958**

(43) Date de publication de la demande:
**06.11.2002 Bulletin 2002/45**

(73) Titulaire: **SANOFI-SYNTHELABO
75013 Paris (FR)**

(72) Inventeurs:
- **SCHOENTJES, Bruno
F-67000 Strasbourg (FR)**
- **SERRADEIL-LE GAL, Claudine
F-31750 Escalquens (FR)**
- **WAGNON, Jean
F-34000 Montpellier (FR)**

(74) Mandataire: **Thouret-Lemaitre, Elisabeth et al
Sanofi-Synthélabo,
Département Brevets,
174, avenue de France
75013 Paris (FR)**

(56) Documents cités:
**WO-A-95/18105**
**WO-A-98/25901**
**US-A- 5 397 801**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 254 134 B1

Printed by Jouve, 75001 PARIS (FR)

## Description

La présente invention a pour objet de nouveaux dérivés de 1,3-dihydro-2*H*-indol-2-one, un procédé pour leur préparation et les compositions pharmaceutiques en contenant.

Les composés selon la présente invention présentent une affinité et une sélectivité pour les récepteurs $V_{1b}$ ou à la fois pour les récepteurs $V_{1b}$ et $V_{1a}$ de l'arginine-vasopressine (AVP).

L'AVP est une hormone connue pour son effet antidiurétique et son effet dans la régulation de la pression artérielle. Elle stimule plusieurs types de récepteurs : $V_1$ ($V_{1a}$,$V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardiovasculaires, hépatiques, pancréatiques, antidiurétiques, agrégants des plaquettes et des effets sur le système nerveux central et périphérique, et sur la sphère utérine.

La localisation des différents récepteurs est décrite dans : S. JARD et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ils ont été clonés chez le rat et l'homme et ils régulent la majorité des effets connus de l'AVP : l'agrégation plaquettaire ; les contractions utérines ; la contraction des vaisseaux ; la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire,...).

Les récepteurs $V_{1b}$ ont été initialement identifiés dans l'adénohypophyse de différentes espèces animales (rat, porc, boeuf, mouton...) y compris chez l'homme (S. JARD et al., Mol. Pharmacol., 1986, 30, 171-177 ; Y. ARSENIJEVIC et al., J. Endocrinol., 1994, 141, 383-391 ; J. SCHWARTZ et al., Endocrinology, 1991, 129 (2), 1107-1109 ; Y. DE KEYSER et al., FEBS Letters, 1994, 356, 215-220) où ils stimulent la libération d'hormone adrénocorticotrophique par l'AVP et potentialisent les effets du CRF sur la libération d'ACTH (G.E. GILLIES et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. DE KEYSER, FEBS Letters, 1994, 356, 215-220 ; T. SUGIMOTO et al., J. Biol. Chem., 1994, 269 (43), 27088-27092 ; M. SAITO et al., Biochem. Biophys. Res. Commun., 1995, 212 (3), 751-757 ; S.J. LOLAIT et al., Neurobiology, 1996, 92, 6783-6787 ; M.A. VENTURA et al., Journal of Molecular endocrinology, 1999, 22, 251-260) et différentes études (hybridation *in situ*, PCR, de l'anglais Polymerase Chain Reaction, ...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régulait le pancréas endocrine en stimulant la sécrétion d'insuline et de glucagon (B. LEE et al., Am. J. Physiol. 269 (Endocrinol. Metab. 32) : E1095-E1100, 1995) ou la production de catécholamines dans la medullo-surrénale qui est le siège d'une synthèse locale d'AVP (E. GRAZZINI et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétants de l'AVP et induisant de ce fait une production soutenue de catécholamines à l'origine d'hypertension résistantes aux antagonistes des récepteurs de l'angiotensine II et aux inhibiteurs de l'enzyme de conversion. Le cortex surrénalien est aussi riche en récepteurs $V_{1a}$ impliqués dans la production de gluco- et minéralo-corticoides (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1b}$ et/ou $V_{1a}$ portés par les cellules de la médulla (G. MAZZOCCHI et al., Peptides, 1997, 18 (2), 191-195 ; E. GRAZZINI et al., J. Clin. Endocrinol. Metab., 1999, 84 (6), 2195-2203).

Les récepteurs $V_{1b}$ sont aussi considérés comme un marqueur des tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroidiens, induisant un syndrome de Cushing dans certains cas (J. BERTHERAT et al., Eur. J. Endocrinol., 1996, 135, 173 ; G.A. WITTERT et al., Lancet, 1990, 335, 991-994 ; G. DICKSTEIN et al., J. Clin. Endocrinol. Metab., 1996, 81 (8), 2934-2941). Les récepteurs $V_{1a}$ sont, quant à eux, un marqueur plus spécifique des cancers pulmonaires à petites cellules (SCLC) (P.J. WOLL et al., Biochem. Biophys. Res. Commun., 1989, 164 (1), 66-73). Ainsi, les composés selon la présente invention sont des outils de diagnostic évidents et offrent une approche thérapeutique nouvelle dans la prolifération et la détection de ces tumeurs, à un stade même précoce (radiomarquage ; SPECT, de l'anglais Single Photon Emission Computed Tomography ; PET Scan, de l'anglais Positron Emission Tomography Scanner).

La présence abondante du messager des récepteurs $V_{1b}$ au niveau stomacal et intestinal, suggère une implication de l'AVP via ce récepteur sur la libération d'hormones gastrointestinales comme la cholécystokinine, la gastrine ou la sécrétine (T. SUGIMOTO et al., Molecular cloning and functional expression of $V_{1b}$ receptor gene, dans Neurohypophysis : Recent Progress of Vasopressin and Oxytocin Research ; T. SAITO, K. KUROKAWA and S. YOSHIDA ed., Elvesier Science, 1995, 409-413).

Des dérivés de 1,3-dihydro-2*H*-indol-2-one ont été décrits dans certaines demandes de brevet comme des ligands des récepteurs de l'arginine-vasopressine et/ou de l'ocytocine : on peut citer les demandes de brevets WO 93/15051, EP 636608, EP 636609, WO 95/18105, WO 97/15556 et WO 98/25901.

A ce jour, aucun composé non peptidique ayant une affinité et une sélectivité pour les récepteurs $V_{1b}$ ou à la fois pour les récepteurs $V_{1b}$ et $V_{1a}$ de l'arginine-vasopressine n'est connu.

On a maintenant trouvé de nouveaux dérivés de 1,3-dihydro-2*H*-indol-2-one qui présentent une affinité et une sélectivité pour les récepteurs $V_{1b}$ ou à la fois pour les récepteurs $V_{1b}$ et $V_{1a}$ de l'arginine-vasopressine.

Ces composés peuvent être utilisés pour la préparation de médicaments utiles dans le traitement ou la prévention de toute pathologie où l'arginine-vasopressine et/ou les récepteurs $V_{1b}$ ou à la fois les récepteurs $V_{1b}$ et les récepteurs $V_{1a}$ sont impliqués, notamment dans le traitement ou la prévention des affections du système cardiovasculaire, par exemple l'hypertension ; du système nerveux central par exemple, le stress, l'anxiété, la dépression, le trouble obsessionnel-compulsif, les attaques de panique ; du système rénal ; du système gastrique ainsi que dans le traitement des cancers pulmonaires à petites cellules ; de l'obésité ; du diabète de type II ; de la résistance à l'insuline ; de l'hypertriglycéridémie ; de l'athérosclérose ; du syndrome de Cushing ; de toutes pathologies consécutives au stress et des états de stress chroniques.

Ainsi, selon un de ses aspects, la présente invention a pour objet des composés de formule :

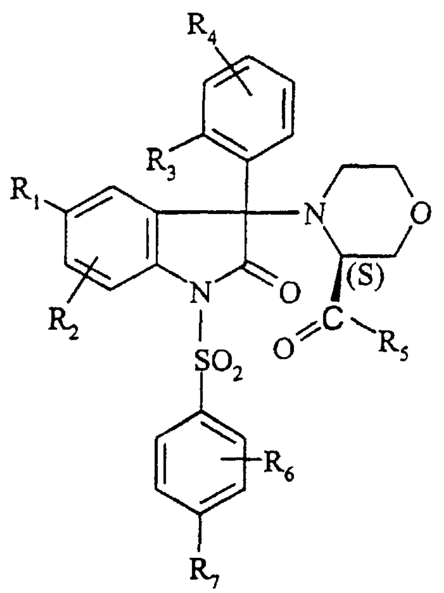

(I)

dans laquelle :

- $R_1$ représente un atome d'halogène ; un $(C_1-C_4)$alkyle ; un $(C_1-C_4)$alcoxy ; un radical trifluorométhyle ; un radical trifluorométhoxy ;
- $R_2$ représente un atome d'hydrogène ; un atome d'halogène ; un $(C_1-C_4)$alkyle ; un $(C_1-C_4)$alcoxy ; un radical trifluorométhyle ;
- ou bien $R_2$ est en position -6- du cycle indol-2-one et $R_1$ et $R_2$ ensemble représentent le radical bivalent triméthylène ;
- $R_3$ représente un atome d'halogène ; un hydroxy ; un $(C_1-C_2)$alkyle ; un $(C_1-C_2)$alcoxy ; un radical trifluorométhoxy ;
- $R_4$ représente un atome d'hydrogène ; un atome d'halogène ; un $(C_1-C_2)$alkyle ; un $(C_1-C_2)$alcoxy ;

- ou bien $R_4$ est en position -3- du phényle et $R_3$ et $R_4$ ensemble représentent le radical méthylènedioxy;
- $R_5$ représente un groupe éthylamino ; un groupe diméthylamino ; un radical azétidin-1-yle ; un $(C_1-C_2)$alcoxy ;
- $R_6$ représente un $(C_1-C_4)$alcoxy ;
- $R_7$ représente un $(C_1-C_4)$alcoxy ; ainsi que leurs solvats et/ou hydrates.

Par atome d'halogène on entend un atome de chlore, de brome, de fluor ou d'iode. Par alkyle, ou respectivement alcoxy, on entend un radical alkyle, ou respectivement un radical alcoxy, linéaire ou ramifié.
Les composés de formule (I) comprennent au moins 2 atomes de carbone asymétriques, l'atome de carbone portant le substituant $COR_5$ a la configuration (S). Les isomères optiquement purs des composés de formule (I) et leurs mélanges en proportions quelconques font partie de l'invention.
Selon la présente invention, on préfère les composés de formule (I) dans laquelle :

- $R_1$ représente un atome d'halogène ; un $(C_1-C_4)$alkyle ; un radical trifluorométhyle ; un radical trifluorométhoxy ;
- $R_2$ représente un atome d'hydrogène ; un atome d'halogène ; un $(C_1-C_4)$alkyle ; un $(C_1-C_4)$alcoxy ; un radical trifluorométhyle ;
- ou bien $R_2$ est en position -6- du cycle indol-2-one et $R_1$ et $R_2$ ensemble représentent le radical bivalent triméthylène ;
- $R_3$ représente un atome d'halogène ; un hydroxy ; un $(C_1-C_2)$alcoxy ;
- $R_4$ représente un atome d'hydrogène ; un atome d'halogène ; un $(C_1-C_2)$alkyle ; un $(C_1-C_2)$alcoxy ;
- ou bien $R_4$ est en position -3- du phényle et $R_3$ et $R_4$ ensemble représentent le radical méthylènedioxy ;
- $R_5$ représente un groupe éthylamino ; un groupe diméthylamino ; un radical azétidin-1-yle ; un $(C_1-C_2)$alcoxy ;
- $R_6$ représente un $(C_1-C_4)$alcoxy ;
- $R_7$ représente un $(C_1-C_4)$alcoxy ; ainsi que leurs solvats et/ou hydrates.

Selon la présente invention, on préfère les composés de formule (I) dans laquelle $R_1$ représente un atome de chlore, un radical méthyle ou un radical trifluorométhoxy.
Selon la présente invention, on préfère les composés de formule (I) dans laquelle $R_2$ représente un atome d'hydrogène ou est en position -6- de l'indol-2-one et représente un atome de chlore, un radical méthyle, un radical méthoxy ou un radical trifluorométhyle.
Selon la présente invention, on préfère les composés de formule (I) dans laquelle $R_3$ représente un atome de chlore ou un radical méthoxy.
Selon la présente invention, on préfère les composés de formule (I) dans laquelle $R_4$ représente un atome d'hydrogène ou est en position -3- du phényle et représente un radical méthoxy.
Selon la présente invention, on préfère les composés de formule (I) dans laquelle $R_5$ représente un groupe diméthylamino, un radical azétidin-1-yle ou un radical méthoxy.
Selon la présente invention, on préfère les composés de formule (I) dans laquelle $R_6$ est en position -2- du phényle et représente un radical méthoxy.
Selon la présente invention, on préfère les composés de formule (I) dans laquelle $R_7$ représente un radical méthoxy.
Plus particulièrement, on préfère les composés de formule (I) dans laquelle :

- $R_1$ représente un atome de chlore ou un radical méthyle ;
- $R_2$ représente un atome d'hydrogène ou est en position -6- de l'indol-2-one et représente un atome de chlore ou un radical méthyle ;
- $R_3$ représente un radical méthoxy ;
- $R_4$ représente un atome d'hydrogène ;
- $R_5$ représente un groupe diméthylamino ;
- $R_6$ est en position -2- du phényle et représente un radical méthoxy ;
- $R_7$ représente un radical méthoxy ;

ainsi que leurs solvats et/ou hydrates.
Selon la présente invention, on préfère les composés de formule (I) sous forme d'isomères optiquement purs, l'atome de carbone en position 3 de l'indol-2-one a soit la configuration (R), soit la configuration (S).
Tout particulièrement, on préfère l'isomère lévogyre des composés de formule (I).
Les composés suivants :

- (3S)-4-[5-Chloro-1-[(2,4-diméthoxyphényl)sulfonyl]-3-(2-méthoxyphényl)-2-oxo-2,3-dihydro-1*H*-indol-3-yl]-N,N-diméthylmorpholine-3-carboxamide, isomère lévogyre ;

- (3S)-4-[6-Chloro-1-[(2,4-diméthoxyphényl)sulfonyl]-3-(2-méthoxyphényl)-5-méthyl-2-oxo-2,3-dihydro-1*H*-indol-3-yl]-N,N-diméthylmorpholine-3-carboxamide, isomère lévogyre ;
(3S)-4-[5-Chloro-1-[(2,4-diméthoxyphényl)sulfonyl]-3-(2-méthoxyphényl)-6-méthyl-2-oxo-2,3-dihydro-1*H*-indol-3-yl]-N,N-diméthylmorpholine-3-carboxamide, isomère lévogyre.

ainsi que leurs solvats et/ou hydrates sont plus particulièrement préférés.

Selon un autre de ses aspects, la présente invention a pour objet un procédé de préparation des composés de formule (I), de leurs solvats et/ou leurs hydrates caractérisé en ce que :

on fait réagir, en présence d'une base, un composé de formule :

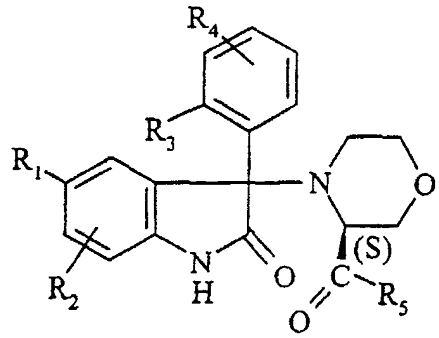
(II)

dans laquelle $R_1$, $R_2$, $R_3$, $R_4$ et $R_5$ sont tels que définis pour un composé de formule (I), avec un halogénure de formule :.

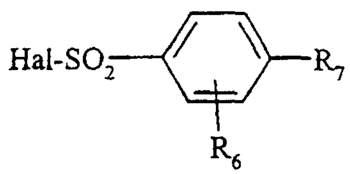
(III)

dans laquelle $R_6$ et $R_7$ sont tels que définis pour un composé de formule (I) et Hal représente un atome d'halogène.

La réaction s'effectue en présence d'une base forte comme un hydrure métallique tel que l'hydrure de sodium ou un alcoolate alcalin tel que le *tert*-butylate de potassium, dans un solvant anhydre tel que le N,N-diméthylformamide ou le tétrahydrofurane et à une température comprise entre -70°C et +60°C. La réaction s'effectue de préférence en utilisant un composé de formule (III) dans laquelle Hal = Cl.

Les composés de formule (I) ainsi obtenus peuvent être ultérieurement séparés du milieu réactionnel et purifiés selon les méthodes classiques, par exemple par cristallisation ou chromatographie.

Les composés de formule (II) se préparent par réaction d'un composé 3-halogéno-1,3-dihydro-2*H*-indol-2-one de formule :

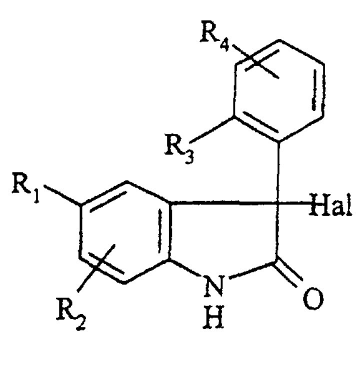
(IV)

dans laquelle $R_1$, $R_2$, $R_3$ et $R_4$ sont tels que définis pour un composé de formule (I) et Hal représente un atome d'halogène, de préférence le chlore ou le brome, avec un composé de formule :

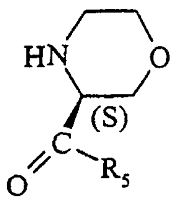

(V)

dans laquelle $R_5$ est tel que défini pour un composé de formule (I). La réaction s'effectue en présence d'une base telle que la diisopropyléthylamine ou la triéthylamine, dans un solvant inerte tel que le dichlorométhane ou le tétrahydrofurane ou un mélange de ces solvants et à une température comprise entre la température ambiante et la température de reflux du solvant.

Les composés de formule (III) sont connus ou préparés par des méthodes connues telles que celles décrites dans EP-0 469 984 B et WO 95/18105. Par exemple, les composés de formule (III) peuvent être préparés par halogénation des acides benzènesulfoniques correspondants ou de leurs sels, par exemple de leurs sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant inerte tel qu'un hydrocarbure halogéné ou le N,N-diméthylformamide et à une température comprise entre -10°C et 200°C.

Le chlorure de 2,4-diméthoxybenzènesulfonyle est préparé selon J. Am. Chem. Soc.,1952, 74, 2008. Le chlorure de 3,4-diméthoxybenzènesulfonyle est commercial, ou préparé selon J. Med. Chem., 1977, 20 (10), 1235-1239.

Les composés de formule (IV) sont connus et se préparent selon des méthodes connues telles que celles décrites dans WO 95/18105.

Par exemple, on transforme un composé de formule :

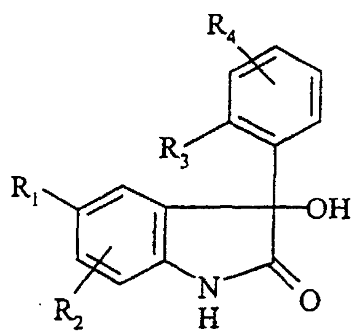

(VI)

dans laquelle $R_1$, $R_2$, $R_3$ et $R_4$ sont tels que définis pour un composé de formule (I), en un composé de formule (IV) dans laquelle Hal = Cl par action du chlorure de thionyle en présence d'une base telle que la pyridine, dans un solvant inerte tel que le dichlorométhane et à une température comprise entre 0°C et la température ambiante.

Selon un autre exemple de préparation des composés de formule (IV), on transforme un composé de formule :

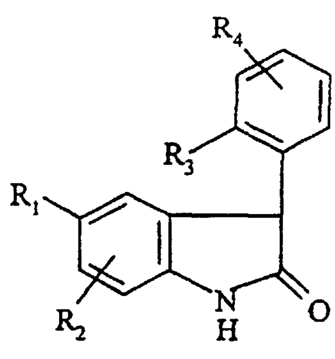

(VII)

dans laquelle $R_1$, $R_2$, $R_3$ et $R_4$ sont tels que définis pour un composé de formule (I), en un composé de formule (IV)

au moyen d'un agent d'halogénation comme le brome selon le procédé décrit dans Farm. Zh.(Kiev), 1976, 5, 30-33.

Les composés de formule (VI) sont connus et se préparent selon des méthodes connues telles que celles décrites dans WO 95/18105.

Par exemple, on prépare un composé de formule (VI) par réaction d'un dérivé de 1*H*-indole-2,3-dione de formule :

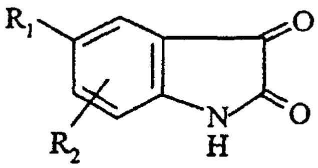

(VIII)

dans laquelle $R_1$ et $R_2$ sont tels que définis pour un composé de formule (I), avec un dérivé organomagnésien de formule :

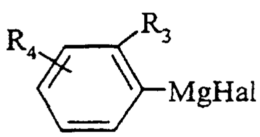

(IX)

dans laquelle $R_3$ et $R_4$ sont tels que définis pour un composé de formule (I) et Hal représente un atome d'halogène, de préférence le brome ou l'iode, dans un solvant inerte tel que le tétrahydrofurane ou l'éther diéthylique et à une température comprise entre 0°C et la température de reflux du solvant.

On peut également préparer un composé de formule (VI) dans laquelle $R_3$ est tel que défini pour un composé de formule (I) et $R_4$, différent de l'hydrogène, est en position -3- ou -6- du phényle, par réaction d'un composé de formule :

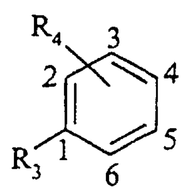

(XVI)

dans laquelle $R_3$ est tel que défini pour un composé de formule (I) et $R_4$ est en position -2- ou -5- du phényle, avec un dérivé du lithium tel que le n-butyllithium, puis l'intermédiaire lithié ainsi obtenu est mis en réaction avec un composé de formule (VIII). La réaction s'effectue dans un solvant tel que l'éther diéthylique, le tétrahydrofurane, l'hexane ou un mélange de ces solvants, à une température comprise entre -70°C et la température ambiante.

Les dérivés de 1*H*-indole-2,3-dione (VIII) sont commerciaux ou se préparent selon les méthodes décrites dans Tetrahedron Letters, 1998, 39, 7679-7682 ; Tetrahedron Letters, 1994, 35, 7303-7306 ; J. Org. Chem., 1977, 42 (8), 1344-1348 ; J. Org. Chem., 1952, 17, 149-156 : J. Am. Chem. Soc., 1946, 68, 2697-2703 ; Organic Syntheses, 1925, V, 71-74 et Advances in Heterocyclic Chemistry, A.R. Katritzky and A.J. Boulton, Academic Press, New-York, 1975, 18, *2-58.*

Les dérivés organomagnésiens (IX) sont préparés selon les méthodes classiques bien connues de l'homme de l'art.

On peut également préparer un composé de formule (VI), par oxydation par l'air d'un composé de formule (VII) en présence d'une base telle que l'hydrure de sodium et en présence de diméthyldisulfure.

De façon particulière, on peut préparer les composés de formule (VI) dans laquelle $R_3$ = $(C_1-C_2)$alcoxy et $R_4$ = H, ou bien $R_3$ = $R_4$ = $(C_1-C_2)$alcoxy avec $R_4$ en position -3 ou -6 du phényle, $R_2$ est différent d'un atome d'halogène et $R_1$ est tel que défini pour un composé de formule (I), en suivant le procédé décrit dans le SCHEMA 1.

SCHEMA 1

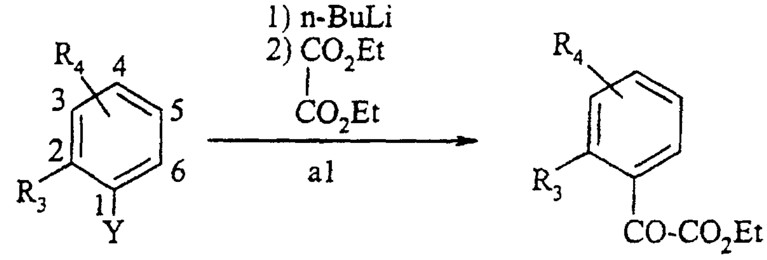


(X) : $R_3$ = $(C_1\text{-}C_2)$alcoxy, $R_4$ = H ; (XI)

$R_3$ = $R_4$ = $(C_1\text{-}C_2)$alcoxy avec

$R_4$ en position -3 ou -6 ;

Y = H ou Br.

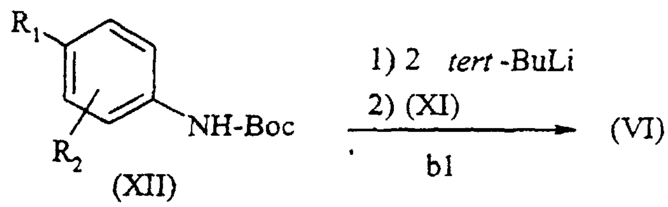


(XII)

A l'étape a1 du SCHEMA 1, on fait d'abord réagir un composé de formule (X) avec un dérivé du lithium tel que le n-butyllithium, en l'absence ou en présence d'une base telle que le N,N,N',N'-tétraméthylènediamine, puis l'intermédiaire lithié ainsi obtenu est mis en réaction avec l'oxalate de diéthyle pour donner le composé de formule (XI). La réaction s'effectue dans un solvant inerte tel que l'éther diéthylique ou le tétrahydrofurane et à une température comprise entre -70°C et la température ambiante.

A l'étape b1, on fait d'abord réagir un composé de formule (XII) avec deux équivalents d'un dérivé du lithium tel que le *tert*-butyllithium, puis l'intermédiaire lithié ainsi obtenu est mis en réaction avec le composé de formule (XI) pour donner le composé de formule (VI) attendu. La réaction s'effectue dans un solvant inerte tel que l'éther diéthylique ou le tétrahydrofurane et à une température comprise entre -70°C et la température ambiante.

Les composés de formule (X) sont commerciaux ou synthétisés de manière classique.

Les composés de formule (XII) sont préparés par réaction des dérivés de l'aniline correspondants avec du di-tert-butyldicarbonate selon les méthodes classiques.

Les composés de formule (VII) sont connus et se préparent selon des méthodes connues telles que celles décrites dans WO 95/18105 ou dans J. Org. Chem., 1968, 33, 1640-1643.

Les composés de formule (V) sont connus ou se préparent selon des méthodes connues. Ainsi, par exemple, les composés de formule (V) dans laquelle $R_5$ représente un groupe éthylamino, diméthylamino ou un radical azétidin-1-yle se préparent selon le SCHEMA 2 ci-après dans lequel Pr représente un groupe N-protecteur, en particulier le benzyle ou le *tert*-butoxycarbonyle, et R représente un $(C_1\text{-}C_2)$alkyle.

SCHEMA 2

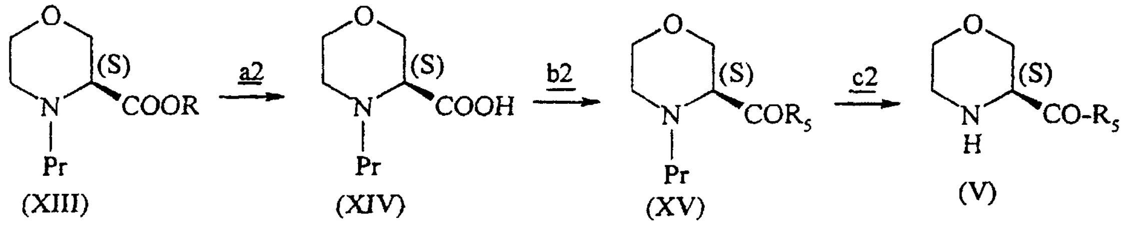


(XIII) (XIV) (XV) (V)

A l'étape a2 du SCHEMA 1, on saponifie un ester de formule (XIII) selon les méthodes classiques, pour obtenir un acide de formule (XIV).

L'acide (XIV) est mis en réaction à l'étape b2 avec l'éthylamine, la diméthylamine ou l'azétidine selon les

méthodes classiques du couplage peptidique pour donner le composé (XV), qui est déprotégé à l'étape c2, selon les méthodes connues, pour donner un composé de formule (V) attendu.

On peut également préparer un composé de formule (XIV) par protection, selon les méthodes classiques, de l'acide (3S)-morpholine-3-carboxylique; ce dernier se prépare selon le procédé décrit dans Bull. Chem. Soc. Jpn, 1987, 60 (8), 2963-2965.

Les composés de formule (V) dans laquelle $R_5$ représente un $(C_1-C_2)$alcoxy s'obtiennent par diverses méthodes connues, en particulier, par déprotection des composés de formule (XIII).

Les composés de formule (XIII) chiraux sont préparés à partir de la (S)-sérine selon le procédé décrit dans J. Chem. Soc. Perkin Trans I, 1985, 2577-2580.

Lorsque l'on souhaite préparer un composé de formule (I) optiquement pur, on fait réagir, de préférence, un composé de formule (II) optiquement pur avec un composé de formule (III) selon le procédé de l'invention.

Les composés de formule (II) optiquement purs se préparent par réaction du composé de formule (IV) racémique avec un composé de formule (V) optiquement pur, puis séparation du mélange des diastéréoisomères selon les méthodes classiques, par exemple par cristallisation ou chromatographie.

Alternativement, on peut faire réagir le mélange des diastéréoisomères du composé de formule (II) avec le composé de formule (III) et séparer le mélange des diastéréoisomères du composé de formule (I) ainsi obtenu.

Au cours de l'une quelconque des étapes de préparation des composés de formule (I) ou des composés intermédiaires de formule (II), (IV), (V) ou (VI), il peut être nécessaire et/ou souhaitable de protéger les groupes fonctionnels réactifs ou sensibles, tels que les groupes amine, hydroxyle ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973, dans Protective Groups in Organic Synthesis, T.W. Greene et P.G.M. Wutts, Ed. John Wiley et sons, 1991 ou dans Protecting Groups, Kocienski P.J., 1994, Georg Thieme Verlag. L'élimination des groupes protecteurs peut s'effectuer à une étape ultérieure opportune en utilisant les méthodes connues de l'homme de l'art et qui n'affectent pas le reste de la molécule concernée.

Les groupes N-protecteurs éventuellement utilisés sont les groupes N-protecteurs classiques bien connus de l'homme de l'art tels que par exemple le groupe *tert*-butoxycarbonyle, fluorénylméthoxycarbonyle, benzyle, benzhydrylidène ou benzyloxycarbonyle.

Les composés de formule (II) sont nouveaux et font partie de l'invention.

Ainsi, selon un autre de ses aspects, l'invention a pour objet des composés de formule :

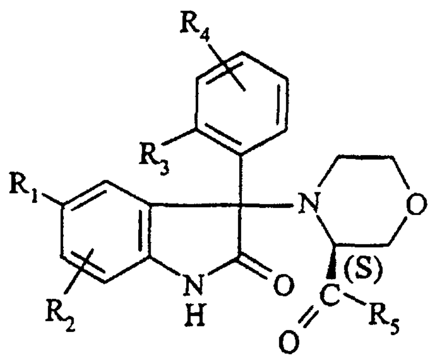

(II)

dans laquelle :

- $R_1$ représente un atome d'halogène ; un $(C_1-C_4)$alkyle ; un $(C_1-C_4)$alcoxy ; un radical trifluorométhyle ; un radical trifluorométhoxy ;
- $R_2$ représente un atome d'hydrogène ; un atome d'halogène ; un $(C_1-C_4)$alkyle ; un $(C_1-C_4)$alcoxy ; un radical trifluorométhyle ;
- ou bien $R_2$ est en position -6- du cycle indol-2-one et $R_1$ et $R_2$ ensemble représentent le radical bivalent triméthylène ;
- $R_3$ représente un atome d'halogène ; un hydroxy ; un $(C_1-C_2)$alkyle ; un $(C_1-C_2)$alcoxy ; un radical trifluorométhoxy ;
- $R_4$ représente un atome d'hydrogène ; un atome d'halogène ; un $(C_1-C_2)$alkyle ; un $(C_1-C_2)$alcoxy ;
- ou bien $R_4$ est en position -3- du phényle et $R_3$ et $R_4$ ensemble représentent le radical méthylènedioxy ;
- $R_5$ représente un groupe éthylamino ; un groupe diméthylamino ; un radical azétidin-1-yle ; un $(C_1-C_2)$alcoxy ;

ainsi que leurs sels avec des acides minéraux ou organiques, sous forme d'isomères optiquement purs ou sous forme de mélange de diastéréoisomères.

Les sels des composés de formule (II) comprennent ceux avec des acides minéraux ou organiques qui permettent une séparation ou une cristallisation convenable des composés de formule (II) tels que le chlorhydrate, le bromhydrate, l'oxalate, le maléate, le succinate, le fumarate, le citrate, l'acétate.

Les composés de formule (I) ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'études biochimiques.

L'affinité des composés de formule (I) selon l'invention pour les récepteurs $V_{1b}$ de l'arginine-vasopressine a été déterminée *in vitro* en utilisant la méthode décrite par Y. DE KEYSER et al., Febs Letters, 1994, 356, 215-220. Cette méthode consiste à étudier *in vitro* le déplacement de l'arginine-vasopressine tritiée ([$^3$H]-AVP) aux récepteurs $V_{1b}$ présents sur des préparations membranaires adénohypophysaires ou cellulaires portant les récepteurs $V_{1b}$ de rat ou humains. Les concentrations inhibitrices de 50 % ($CI_{50}$) de la fixation de l'arginine-vasopressine tritiée des composés selon l'invention sont faibles et varient de $10^{-6}$ à $10^{-9}$M, plus particulièrement $10^{-8}$M.

L'affinité des composés de formule (I) selon l'invention pour les récepteurs $V_{1a}$ de l'arginine-vasopressine a été déterminée *in vitro* en utilisant la méthode décrite par M. THIBONNIER et al., J. Biol. Chem., 1994, 269, 3304-3310. Cette méthode consiste à étudier *in vitro* le déplacement de l'arginine-vasopressine tritiée ([$^3$H]-AVP) aux récepteurs $V_{1a}$ présents sur des préparations membranaires ou cellulaires portant les récepteurs $V_{1a}$ de rat ou humains. Parmi les composé de formule (I), certains présentent aussi une affinité pour les récepteurs $V_{1a}$ de l'arginine-vasopressine avec des $CI_{50}$ qui varient de $10^{-6}$ à $10^{-9}$M, plus particulièrement $10^{-7}$M.

L'affinité des composés de formule (I) selon l'invention pour les récepteurs $V_2$ de la vasopressine a également été étudiée (méthode décrite par M. Bimbaumer et al., Nature (Lond.), 1992, 357, 333-335). Les composés étudiés sont peu ou pas affins pour les récepteurs $V_2$.

Les composés de la présente invention sont notamment des principes actifs de compositions pharmaceutiques, dont la toxicité est compatible avec leur utilisation en tant que médicaments.

Selon un autre de ses aspects, la présente invention concerne l'utilisation des composés de formule (I), de leurs solvats et/ou hydrates pharmaceutiquement acceptables, pour la préparation de médicaments destinés à traiter toute pathologie où l'arginine-vasopressine et/ou ses récepteurs $V_{1b}$ ou à la fois ses récepteurs $V_{1b}$ et ses récepteurs $V_{1a}$ sont impliqués.

Selon un autre de ses aspects, la présente invention concerne l'utilisation des composés de formule (I), de leurs solvats et/ou hydrates pharmaceutiquement acceptables pour la préparation de médicaments destinés à traiter les pathologies du système cardiovasculaire, du système nerveux central, du système rénal, du système gastrique ainsi que les cancers du poumon à petites cellules, l'obésité, le diabète de type II, la résistance à l'insuline, l'hypertriglycéridémie, l'athérosclérose, le syndrome de Cushing, toutes pathologies consécutives au stress et les états de stress chroniques.

Ainsi les composés selon l'invention peuvent être utilisés, chez l'homme ou chez l'animal, dans le traitement ou la prévention de différentes affections vasopressine-dépendantes tels que les affections cardiovasculaires, comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase ; les affections du système nerveux central comme la migraine, le vasospasme cérébral, l'hémorragie cérébrale, les oedèmes cérébraux, la dépression, l'anxiété, le stress, le trouble obsessionnel-compulsif, les attaques de panique, les états psychotiques, les troubles de la mémoire par exemple ; les affections du système rénal comme le vasospasme rénal, la nécrose du cortex rénal ; le diabète insipide néphrogénique ; les affections du système gastrique comme le vasospasme gastrique, l'hépatocirrhose, les ulcères, la pathologie des vomissements, par exemple la nausée y compris la nausée due à une chimiothérapie, le mal des transports ; la néphropathie diabétique. Les composés selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules ; des encéphalopathies hyponatrémiques ; du syndrome pulmonaire, de la maladie de Meniére ; du glaucome, de la cataracte ; de l'obésité ; du diabète de type II ; de l'athérosclérose ; du syndrome de Cushing ; de la résistance à l'insuline, de l'hypertriglycéridémie, dans les traitements post-opératoires, notamment après une chirurgie abdominale.

Les composés selon l'invention peuvent aussi être utilisés dans le traitement ou la prévention de toutes les pathologies consécutives au stress comme la fatigue et ses syndrômes, les désordres ACTH dépendants, les troubles cardiaques, la douleur, les modifications de la vidange gastrique, de l'excrétion fécale (colite, syndrôme du colon irritable, maladie de Crohn), de la sécrétion acide, l'hyperglycémie, l'immunosuppression, les processus inflammatoires

(arthrite rhumatoïde et ostéoarthrite), les infections multiples, les cancers, l'asthme, le psoriasis, les allergies et les désordres neuropsychiatriques variés tel que l'anorexie nerveuse, la boulimie, les troubles de l'humeur, la dépression, l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysorurrénalien.

Les composés selon l'invention peuvent également être utilisés comme psychostimulants, provoquant l'augmentation de l'éveil, la réactivité émotionnelle face à l'environnement et facilitant l'adaptation.

Les composés de formule (I) ci-dessus, leurs solvats et/ou hydrates pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (I), un de ses solvats et/ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose, la cellulose microcristalline, l'amidon, le phosphate dicalcique, de liants comme par exemple la polyvinylpyrrolidone, l'hydroxypropylméthylcellulose, des délitant comme la polyvinylpyrrolidone réticulée, la carboxyméthylcellulose réticulée, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lequel le principe actif peuvent être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, $\alpha$, $\beta$, $\gamma$-cyclodextrine, 2-hydroxypropyl-$\beta$-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Les compositions de la présente invention peuvent contenir, à côté des composés de formule (I), de leurs solvats et/ou hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur les récepteurs du CRF.

Les composés selon l'invention pourront également être utilisés pour la préparation de compositions à usage vétérinaire.

Les PREPARATIONS et EXEMPLES suivants illustrent l'invention sans toute fois la limiter.

Dans les Préparations et dans les Exemples on utilise les abréviations suivantes :

éther : éther diéthylique
éther iso : éther diisopropylique
DMF : N,N-diméthylformamide
THF : tétrahydrofurane
DCM : dichlorométhane
AcOEt : acétate d'éthyle
DSPEA : diisopropyléthylamine
TFA : acide trifluoroacétique
Boc : *tert*-butoxycarbonyle
Cbz : benzyloxycarbonyle
BOP : benzotriazol-1-yloxytris(diméthylamino)phosphonium hexafluorophosphate
PyBOP : benzotriazol-1-yloxytripyrrolidinophosphonium hexafluorophosphate
DCC : 1,3-dicyclohexylcarbodiimide
HOBT : 1-hydroxybenzotriazole hydrate
F : point de fusion
TA : température ambiante
Eb : température d'ébullition
CLHP : chromatographie liquide haute performance.

Les spectres de résonance magnétique du proton (RMN $^{1}H$) sont enregistrés à 200 MHz dans du DMSO-$d_6$, en utilisant le pic du DMSO-$d_6$ comme référence. Les déplacements chimiques δ sont exprimés en parties par million (ppm). Les signaux observés sont exprimés ainsi : s: singulet ; se : singulet élargi ; d : doublet ; d.d : doublet dédoublé ; t : triplet ; q : quadruplet ; m : massif ; mt : multiplet.

Les spectres de masse indiquent la valeur $MH^+$.

PREPARATIONS

Préparations des composés de formule (IV).

Préparation 1.1

3,5-Dichloro-3-(2-méthoxyphényl)-1,3-dihydro-2*H*-indol-2-one.

(IV) : $R_1$ = Cl ; $R_2$ = H ; $R_3$ = OCH3 ; $R_4$ = H ; Hal = Cl.

A) 5-Chloro-3-hydroxy-3-(2-méthoxyphényl)-1,3-dihydro-2*H*-indol-2-one.

On prépare ce composé selon le mode opératoire décrit dans WO 95/18105. On prépare une solution de bromure de 2-méthoxyphénylmagnésium à partir de 16 g de magnésium dans 35 ml d'éther et d'une solution de 124 g de 1-bromo-2-méthoxybenzène dans 175 ml d'éther. On ajoute, en goutte à goutte, sous atmosphère d'argon, cette solution à un mélange de 30 g de 5-chloro-1*H*-indole-2,3-dione dans 250 ml de THF, préalablement refroidi au bain de glace, puis laisse sous agitation en laissant remonter la température à TA. Après 1 heure d'agitation à TA, on verse lentement le mélange réactionnel sur une solution saturée de $NH_4Cl$ et évapore le THF sous vide. On essore le précipité formé et le lave à l'éther iso. On obtient 42 g du produit attendu que l'on utilise tel quel à l'étape suivante.

B) 3,5-Dichloro-3-(2-méthoxyphényl)-1,3-dihydro-2*H*-indol-2-one.

On prépare ce composé selon le mode opératoire décrit dans WO 95/18105. On refroidit à 0°C un mélange de 12,71 g du composé obtenu à l'étape précédente dans 105 ml de DCM, ajoute 5,3 ml de pyridine puis, 4,9 ml de chlorure de thionyle. Après 30 minutes sous agitation, on ajoute de l'eau au mélange réactionnel et évapore le DCM sous vide. On essore le précipité formé, le lave trois fois à l'eau puis trois fois à l'éther iso et le sèche. On obtient 13,66 g du produit attendu que l'on utilise tel quel.

Préparation 1.2

3,6-Dichloro-3-(2-méthoxyphényl)-5-méthyl-1,3-dihydro-2H-indol-2-one.

(IV) : $R_1$ = $CH_3$ ; $R_2$ = 6-Cl ; $R_3$ = $OCH_3$ ; $R_4$ = H ; Hal = Cl.

A) 6-Chloro-5-méthyl-3-méthylthio-1,3-dihydro-2*H*-indol-2-one et 4-chloro-5-méthyl-3-méthylthio-1,3-dihydro-2*H*-indol-2-one.

Dans 320 ml de DCM refroidi à -70°C, on introduit 8,5 ml de chlore, puis ajoute, en 20 minutes et à -70°C, une solution de 24 ml de méthylthioacétate d'éthyle dans 60 ml de DCM et laisse 15 minutes sous agitation à -70°C. On ajoute ensuite, à -70°C et en 30 minutes, une solution de 52,64 g de 3-chloro-4-méthylaniline dans 100 ml de DCM et laisse 1 heure 45 minutes sous agitation à -70°C. On ajoute enfin, à -70°C, 41,3 ml de triéthylamine et laisse 1 heure sous agitation en laissant remonter la température à TA. On lave le mélange réactionnel deux fois par 250 ml d'eau, sèche la phase organique sur $MgSO_4$ et évapore sous vide le solvant. On reprend le résidu dans un mélange de 600 ml d'éther et 130 ml d'HCl 2N et laisse 72 heures sous agitation à TA. On filtre une insoluble, décante le filtrat, lave deux fois la phase organique à l'eau, sèche sur $MgSO_4$ et évapore sous vide le solvant. On chromatographie le résidu sur gel de silice en éluant au DCM puis par le mélange DCM/AcOEt (85/15 ; v/v). On rechromatographie le mélange obtenu sur gel de silice en éluant au DCM puis par le mélange DCM/AcOEt (95/5 ; v/v). On sépare les deux isomères :

- l'isomère le moins polaire qui est le 6-chloro-5-méthyl-3-méthylthio-1,3-dihydro-2*H*-indol-2-one et obtient 1,16 g.
- l'isomère le plus polaire qui est le 4-chloro-5-méthyl-3-méthylthio-1,3-dihydro-2*H*-indol-2-one et obtient 0,72 g.

B) 6-Chloro-5-méthyl-1*H*-indole-2,3-dione.

On chauffe à reflux pendant 1 heure un mélange de 1,16 g de 6-chloro-5-méthyl-3-méthylthio-1,3-dihydro-2*H*-indol-2-one obtenu à l'étape précédente et 0,681 g de N-chlorosuccinimide dans 100 ml de tétrachlorure de carbone. On concentre sous vide le mélange réactionnel, reprend le résidu dans un mélange de 80 ml de THF et 20 ml d'eau puis chauffe à reflux pendant 16 heures. On évapore sous vide le THF, extrait la phase aqueuse restante à l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le gradient du mélange DCM/AcOEt jusqu'à (85/15 ; v/v). On obtient 0,793 g du produit attendu, F = 264°C.

C) 6-Chloro-3-hydroxy-3-(2-méthoxyphényl)-5-méthyl-1,3-dihydro-2*H*-indol-2-one.

On prépare une solution de bromure de 2-méthoxyphénylmagnésium à partir de 0,687 g de magnésium dans 1,5 ml d'éther et d'une solution de 5,35 g de 1-bromo-2-méthoxybenzène dans 7,55 ml d'éther. On ajoute, en goutte à goutte, sous atmosphère d'argon, cette solution à un mélange de 1,4 g du composé obtenu à l'étape précédente dans 14 ml de THF préalablement refroidi au bain de glace, puis laisse sous agitation en laissant remonter la température à TA. Après 1 heure d'agitation à TA, on verse lentement le mélange réactionnel sur une solution saturée de $NH_4Cl$, évapore le THF sous vide, extrait à l'AcOEt, lave la phase organique à l'eau, par une solution saturée de NaCl, sèche sur $Na_2SO_4$ et évapore sous vide l'AcOEt. On chromatographie le résidu sur gel de silice en éluant au DCM puis par le mélange DCM/MeOH (98/2 ; v/v). On obtient 1,6 g du produit attendu après cristallisation dans le mélange THF/MeOH, F = 266°C.

D) 3,6-Dichloro-3-(2-méthoxyphényl)-5-méthyl-1,3-dihydro-2*H*-indol-2-one.

On refroidit au bain de glace une suspension de 1,7 g du composé obtenu à l'étape précédente dans 10 ml de DCM, ajoute 1,4 ml de pyridine puis 1,4 ml de chlorure de thionyle et laisse 4 heures sous agitation à TA. On ajoute de l'eau au mélange réactionnel, après décantation lave la phase organique à l'eau jusqu'à pH = 7, sèche sur $Na_2SO_4$ et évapore sous vide le solvant. On obtient 1,09 g du produit attendu après cristallisation dans le mélange DCM/éther iso.

Préparation 1.3

3,5-Dichloro-3-(2-méthoxyphényl)-6-méthyl-1,3-dihydro-2*H*-indol-2-one.
(IV) : $R_1$ = Cl ; $R_2$ = 6-$CH_3$ ; $R_3$ = $OCH_3$ ; $R_4$ = H ; Hal = Cl.

A) 2-(2-méthoxyphényl)-2-oxoacétate d'éthyle.

On refroidit à -70°C, sous atmosphère d'argon, une solution de 27 g de 1-bromo-2-méthoxybenzène dans 270 ml d'éther, ajoute, goutte à goutte, 90 ml d'une solution 1,6 M de n-butyllithium dans le pentane, puis laisse 45 minutes sous agitation. On ajoute rapidement 78 ml d'oxalate de diéthyle et laisse sous agitation en laissant remonter la température à TA. Après 1 heure d'agitation à TA, on ajoute au mélange réactionnel une solution saturée de $NH_4Cl$, décante, extrait la phase aqueuse à l'éther, lave les phases organiques jointes à l'eau, par une solution saturée de NaCl, sèche sur $Na_2SO_4$ et évapore les solvants sous vide. On élimine l'oxalate de diéthyle en excès par distillation sous vide (Eb = 87°C sous 2000 Pa). On chromatographie le produit résultant sur gel de silice en éluant par le mélange DCM/hexane (50/50 ; v/v) puis au DCM. Le produit obtenu est purifié par distillation sous vide. On obtient 13 g du produit attendu, Eb = 110°C sous 3 Pa.

B) 5-Chloro-3-hydroxy-3-(2-méthoxyphényl)-6-méthyl-1,3-dihydro-2*H*-indol-2-one.

a) 4-Chloro-3-méthylphénylcarbamate de *tert*-butyle.
On laisse 24 heures sous agitation à TA un mélange de 10 g de 4-chloro-3-méthylaniline et 15,26 g de di-*tert*-butyldicarbonate dans 50 ml de dioxane. On concentre sous vide le mélange réactionnel et chromatographie le résidu sur gel de silice en éluant par le gradient du mélange DCM/hexane de (50/50 ; v/v) à (70/30 ; v/v). On obtient 5,6 g du produit attendu que l'on utilise tel quel.

b) On refroidit à -70°C, sous atmosphère d'argon, une solution de 5 g de 4-chloro-3-méthylphénylcarbamate de *tert*-butyle dans 45 ml d'éther, ajoute, goutte à goutte; 30 ml d'une solution 1,5M de *tert*-butyllithium dans le pentane, laisse 1 heure sous agitation en faisant remonter la température à -10°C et laisse 1 heure 45 minutes sous agitation à -10°C. On refroidit le mélange réactionnel à -70°C, ajoute, goutte à goutte, une solution de 5 g du composé obtenu à l'étape A dans 25 ml de THF, laisse 1 heure sous agitation en laissant remonter la température à -30°C

puis une nuit en laissant remonter la température à TA. On ajoute une solution saturée de $NH_4Cl$ au mélange réactionnel, évapore le THF, extrait trois fois la phase aqueuse résultante à l'AcOEt, lave la phase organique à l'eau, par une solution saturée de NaCl, sèche sur $Na_2SO_4$, évapore partiellement le solvant et essore le produit cristallisé. On obtient 2,6 g du produit attendu, F = 254-256°C.

C) 3,5-Dichloro-3-(2-méthoxyphényl)-6-méthyl-1,3-dihydro-2*H*-indol-2-one.

On refroidit à 0°C un mélange de 1,25 g du composé obtenu à l'étape B dans 20 ml de DCM, ajoute 0,51 ml de pyridine puis 0,47 ml de chlorure de thionyle et laisse 1 heure sous agitation après avoir laissé la température remonter à TA. On ajoute de l'eau et du DCM au mélange réactionnel, après décantation lave quatre fois la phase organique à l'eau, sèche sur $Na_2SO_4$ et évapore le solvant sous vide. On obtient 1,4 g du produit attendu.

Préparation des composés de formule (V).

Préparation 2.1

(3S)-N,N-Diméthylmorpholine-3-carboxamide.

(V) : $R_5$ = $-N(CH_3)_2$.

A) (S)-N-Benzylsérine.

A une solution de 30 g de (S)-sérine dans 150 ml d'une solution aqueuse 2M de NaOH, on ajoute, goutte à goutte 28,5 ml de benzaldéhyde et laisse 2 heures sous agitation à TA. On refroidit le mélange réactionnel à 4°C, ajoute, par portions, 3 g de borohydrure de sodium et laisse 1 heure sous agitation à 4°C puis 1 heure à TA. On lave le mélange réactionnel à l'éther, acidifie la phase aqueuse à pH = 6,5 par ajout d'une solution d'HCl 10N et essore le précipité formé. On obtient 20 g du produit attendu après cristallisation dans l'eau.

B) Acide (3S)-4-benzyl-5-oxomorpholine-3-carboxylique.

On refroidit à 4°C un mélange de 20 g du composé obtenu à l'étape précédente dans 100 ml d'eau, ajoute 5 g de NaOH en pastilles puis, en goutte à goutte et en 30 minutes, 10 ml de chlorure de chloroacétyle. On ajoute ensuite 30 ml d'une solution aqueuse à 30 % (poids/poids) de NaOH et chauffe le mélange réactionnel à 30°C pendant 2 heures. On refroidit le mélange réactionnel à 4°C, acidifie à pH = 1 par ajout d'une solution d'HCl 10N et essore le précipité formé. On obtient 9 g du produit attendu.

C) Ester éthylique de l'acide (3S)-4-benzyl-5-oxomorpholine-3-carboxylique.

On prépare ce composé en utilisant la méthode décrite par B. Neises et W. Steglich dans Angew. Chem. Int. Ed. Engl., 1978, <u>17</u> (7), 522-524.

On refroidit à 4°C un mélange de 8 g du composé obtenu à l'étape précédente dans 40 ml de DCM et 2 ml de DMF, ajoute successivement 10 ml d'EtOH, 1 g de 4-diméthylaminopyridine et 15 g de 1,3-dicyclohexylcarbodiimide, puis laisse 18 heures le mélange réactionnel sous agitation à TA. On filtre un insoluble et concentre sous vide le filtrat. On reprend le résidu au DCM et filtre à nouveau un insoluble. On lave la phase organique résultante par une solution aqueuse à 5 % de $KHSO_4$, par une solution aqueuse à 5 % de $NaHCO_3$, sèche sur $Na_2SO_4$ et évapore sous vide le solvant. On chromatographie le résidu sur gel de silice en éluant par le mélange DCM/AcOEt (95/5 ; v/v). On obtient 8 g du produit attendu.

D) Ester éthylique de l'acide (3S)-4-benzylmorpholine-3-carboxylique.

On refroidit à 4°C, sous atmosphère d'azote, une solution de 7,8 g du composé obtenu à l'étape précédente dans 80 ml de THF anhydre, ajoute 22 ml d'une solution 2M de complexe borane-diméthylsulfure dans le THF et laisse le mélange réactionnel sous agitation en laissant remonter la température à TA. Après 3 heures d'agitation à TA, on ajoute de l'eau jusqu'à la fin du dégagement gazeux. On évapore sous vide le THF, alcalinise la phase aqueuse résultante à pH = 10 par ajout de NaOH en pastilles, extrait à l'éther, sèche la phase organique sur $MgSO_4$ et évapore sous vide le solvant. On chromatographie le résidu sur gel de silice en éluant par le mélange DCM/AcOEt (99/1 ; v/v). On obtient 4,7 g du produit attendu.

E) Acide (3S)-4-benzylmorpholine-3-carboxylique.

A une solution de 4,5 g du composé obtenu à l'étape précédente dans 20 ml d'EtOH, on ajoute à TA 7 ml

d'une solution aqueuse à 50 % de NaOH et laisse 3 heures sous agitation à TA. On acidifie le mélange réactionnel à pH = 2 par ajout d'une solution d'HCl concentrée et concentre sous vide. Le produit résultant est utilisé tel quel à l'étape suivante.

F) (3S)-4-Benzyl-N,N-diméthylmorpholine-3-carboxamide.

A un mélange du composé obtenu à l'étape précédente dans 20 ml de DCM, on ajoute à TA 9,81 g de PyBOP puis 10 ml de DIPEA et laisse 30 minutes sous agitation à TA. Puis on ajoute de la diméthylamine gaz par barbottage pendant 10 minutes et laisse 18 heures sous agitation à TA. On concentre sous vide le mélange réactionnel, extrait le résidu à l'AcOEt, lave la phase organique par une solution aqueuse à 5 % de $Na_2CO_3$, par une solution saturée de NaCl, sèche sur $MgSO_4$ et concentre sous vide le solvant. On chromatographie le résidu sur gel de silice en éluant par le mélange DCM/MeOH (98/2 ; v/v). On obtient 1,85 g du produit attendu.

G) (3S)-N,N-Diméthylmorpholine-3-carboxamide.

On hydrogène, à 30°C et sous pression atmosphérique, un mélange de 1,83 g du composé obtenu à l'étape précédente et 0,09 g de palladium sur charbon à 10 % dans 50 ml d'EtOH. On filtre le catalyseur sur Célite® et concentre sous vide le filtrat. On obtient 1 g du produit attendu.
RMN $^1H$ : DMSO-$d_6$ : $\delta$ (ppm) : 2,85 : s : 3H ; 3,5 : s : 3H ; 3,2 : mt : 2H ; 3,3 à 4,3 : m : 4H ; 4,6 : mt : 1H.

Préparations des composés de formule (II).

Préparation 3.1

(3S)-4-[5-Chloro-3-(2-méthoxyphényl)-2-oxo-2,3-dihydro-1*H*-indol-3-yl]-N,N-diméthylmorpholine-3-carboxamide, mélange de diastéréoisomères.
(II) : $R_1$ = Cl ; $R_2$ = H ; $R_3$ = $OCH_3$ ; $R_4$ = H ; $R_5$ = $-N(CH_3)_2$.
A une solution de 0,9 g du composé obtenu à la Préparation 2.1 dans 25 ml de DCM, on ajoute à TA 1,75 g du composé obtenu à la Préparation 1.1 puis 0,8 ml de triéthylamine et laisse 48 heures sous agitation à TA. On concentre sous vide le mélange réactionnel, extrait le résidu à l'AcOEt, lave la phase organique par une solution aqueuse à 5 % de $KHSO_4$, par une solution aqueuse à 5 % de $Na_2CO_3$, par une solution saturée de NaCl, sèche sur $MgSO_4$ et évapore sous vide le solvant. On chromatographie le résidu sur gel de silice en éluant par le mélange DCM/MeOH (98/5 ; v/v). On obtient 1,63 g du produit attendu.
RMN $^1H$ : DMSO-$d_6$ : $\delta$ (ppm) : 2,0 à 2,8 : m : 6H ; 3,3 : 2s : 3H ; 3,4 à 4,2 : m : 7H ; 6,4 à 8,0 : mt : 7H ; 10,0 à 10,5 : 2s : 1H.

Préparations 3.2 et 3.3

(3S)-4-[6-Chloro-3-(2-méthoxyphényl)-5-méthyl-2-oxo-2,3-dihydro-1*H*-indol-3-yl]-N,N-diméthylmorpholine-3-carboxamide, isomère A et isomère B.
(II) : $R_1$ = $CH_3$ ; $R_2$ = 6-Cl ; $R_3$ = $OCH_3$ ; $R_4$ = H ; $R_5$ = $-N(CH_3)_2$.
A une solution de 0,62 g du composé obtenu à la Préparation 1.2 dans 40 ml de DCM, on ajoute à TA 1,02 g du composé obtenu à la Préparation 2.1 puis 0,7 ml de triéthylamine et laisse 18 heures sous agitation à TA. On concentre sous vide le mélange réactionnel, extrait le résidu à l'AcOEt, lave la phase organique par une solution aqueuse à 5 % de $KHSO_4$, par une solution aqueuse à 5 % de $Na_2CO_3$, par une solution saturée de NaCl, sèche sur $MgSO_4$ et évapore sous vide le solvant. On chromatographie le résidu sur alumine en éluant par le mélange DCM/MeOH (99/1 ; v/v). On sépare les deux isomères :

- le moins polaire, isomère A : composé de la Préparation 3.2 que l'on cristallise dans le mélange DCM/éther iso et obtient 0,4 g, F = 237°C.
  $\alpha_D^{25}$ = + 106° (c = 0,1 ; chloroforme).
  RMN $^1H$ : DMSO-$d_6$ : $\delta$ (ppm) : 2,1 : se : 3H ; 2,5 à 2,9 : 2s : 6H ; 3,3 à 4,2 : m : 10H ; 6,4 : se : 1H ; 6,7 : se 1H ; 6,8 : d : 1H ; 7,0 : t : 1H ; 7,2 : t : 1H ; 7,9 : d : 1H ; 10,2 : s : 1H.
- le plus polaire, isomère B : composé de la Préparation 3.3 que l'on cristallise dans le mélange DCM/éther iso et obtient 0,73 g, F = 177°C.
  $\alpha_D^{25}$ = - 150° (c = 0,1 ; chloroforme).

RMN $^1H$ : DMSO-$d_6$ : $\delta$ (ppm) : 2,0 : s : 3H ; 2,2 à 2,6 : 2s : 6H ; 3,4 : s : 3H ; 3,5 à 3,8 : mt : 6H ; 4,0 : mt :

1H ; 6,5 : s : 1H ; 6,7 : s : 1H ; 6,8 : d : 1H ; 7,0 : t : 1H ; 7,2 : t : 1H ; 7,8 : d : 1H ; 10,0 : s : 1H.

Préparations 3.4 et 3.5

(3S)-4-[5-Chloro-3-(2-méthoxyphényl)-6-méthyl-2-oxo-2,3-dihydro-1*H*-indol-3-yl]-N,N-diméthylmorpholine-3-carboxamide, isomère A et isomère B.

(II) : $R_1$ = Cl ; $R_2$ = 6-$CH_3$ ; $R_3$ = $OCH_3$ ; $R_4$ = H ; $R_5$ = -$N(CH_3)_2$.

A un mélange de 1,4 g du composé obtenu à la Préparation 1.3 et 0,7 g du composé obtenu à la Préparation 2.1 dans 15 ml de DCM, on ajoute 1,13 g de DIPEA et laisse 3 heures sous agitation à TA. On rajoute 0,65 g de DIPEA, laisse 48 heures sous agitation à TA et observe la formation d'un précipité. On concentre sous vide le mélange réactionnel, reprend le résidu par une solution aqueuse à 5 % de $Na_2CO_3$, extrait deux fois à l'AcOEt, lave la phase organique à l'eau, par une solution saturée de NaCl et essore le précipité présent dans la phase organique correspondant à l'isomère A, composé le moins polaire sur alumine, DCM/MeOH (95/5 ; v/v) (composé de la Préparation 3.4). Les jus d'essorage sont chromatographiés sur alumine en éluant par le mélange DCM/MeOH (95/5). On sépare les deux isomères :

- le moins polaire, isomère A : composé de la Préparation 3.4 que l'on joint au produit obtenu précédemment et cristallise dans le mélange DCM/AcOEt/MeOH pour obtenir 0,292 g, F = 230-238°C.
  $\alpha_D^{25}$ = + 79,6° (c = 0,1 ; chloroforme).
- le plus polaire, isomère B : composé de la Préparation 3.5 et obtient 0,887 g.
  $\alpha_D^{25}$ = - 100° (c = 0,1 ; chloroforme).

EXEMPLES 1 et 2

(3S)-4-[5-Chloro-1-[(2,4-diméthoxyphényl)sulfonyl]-3-(2-méthoxyphényl)-2-oxo-2,3-dihydro-1*H*-indol-3-yl] -N,N-diméthylmorpholine-3-carboxamide, isomère A et isomère B.

(I) : $R_1$ = Cl ; $R_2$ = H ; $R_3$ = $OCH_3$ ; $R_4$ = H ; $R_5$ = -$N(CH_3)_2$ ; $R_6$ = 2-$OCH_3$ ; $R_7$ = $OCH_3$.

On refroidit à 4°C, sous atmosphère d'azote, un mélange de 1,85 g du composé obtenu à la Préparation 3.1 dans 18 ml de DMF, ajoute 0,191 g d'hydrure de sodium à 60 % dans l'huile et laisse 30 minutes sous agitation à 4°C. On ajoute ensuite 1,02 g de chlorure de 2,4-diméthoxybenzènesulfonyle et laisse 3 heures sous agitation à TA. On ajoute 50 ml d'eau au mélange réactionnel, extrait à l'AcOEt, lave la phase organique à l'eau, par une solution saturée de NaCl, sèche sur $MgSO_4$ et évapore sous vide le solvant. On chromatographie le résidu sur gel de silice en éluant par le mélange DCM/MeOH (98/2 ; v/v). On sépare les deux isomères :

- le moins polaire, isomère A : composé de l'Exemple 1 que l'on cristallise dans l'hexane et obtient 0,437 g, F = 156°C.
  $\alpha_D^{25}$ = + 136° (c = 0,1 ; chloroforme).
  RMN $^1H$ : DMSO-$d_6$ : δ (ppm) : 2,1 à 2,7 : m : 8H ; 2,9 : se : 3H ; 3,6 à 4,2 : m+2s : 11H ; 6,7 : m : 4H ; 7,1 : t : 1H ; 7,3 : td : 1H ; 7,4 : dd : 1H ; 7,8 : d : 1H ; 7,9 ; d+m : 2H.
- le plus polaire, isomère B : composé de l'Exemple 2 que l'on cristallise dans le mélange DCM/éther iso et obtient 0,7 g, F = 164°C.
  $\alpha_D^{25}$ = - 204° (c = 0,1 ; chloroforme).

RMN $^1H$ : DMSO-$d_6$ : δ (ppm) : 2,5 : se : 6H ; 3,1 à 3,7 : m+s : 10H ; 3,7 à 4,0 : 2s : 6H ; 6,5 à 6,8 : 2m : 3H ; 6,9 : d : 1H ; 7,1 : t : 1H ; 7,3 : t : 1H ; 7,5 : dd : 1H ; 7,8 : d : 1H ; 7,9 : d : 1H ; 8,1: se : 1H.

EXEMPLE 3

(3S)-4-[6-Chloro-1-[(2,4-diméthoxyphényl)sulfonyl]-3-(2-méthoxyphényl)-5-méthyl-2-oxo-2,3-dihydro-1*H*-indol-3-yl]-N,N-diméthylmorpholine-3-carboxamide, isomère lévogyre.

(I) : $R_1$ = $CH_3$ ; $R_2$ = 6-Cl ; $R_3$ = $OCH_3$ ; $R_4$ = H ; $R_5$ = -$N(CH_3)_2$ ; $R_6$ = 2-$OCH_3$ ; $R_7$ = $OCH_3$.

On refroidit à 4°C, sous atmosphère d'azote, un mélange de 0,61 g du composé obtenu à la Préparation 3.3 (isomère B) dans 5 ml de DMF, ajoute 0,06 g d'hydrure de sodium à 60 % dans l'huile et laisse 30 minutes sous agitation à TA. On ajoute ensuite 0,36 g de chlorure de 2,4-diméthoxybenzènesulfonyle et laisse 2 heures sous agitation à TA. On ajoute 50 ml d'eau au mélange réactionnel, extrait à l'AcOEt, lave la phase organique par une solution aqueuse à 5 % de $Na_2CO_3$, par une solution saturée de NaCl, sèche sur $Na_2SO_4$ et évapore sous vide le solvant. On chromatographie le résidu sur gel de silice en éluant par le mélange DCM/AcOEt (70/30 ; v/v). On obtient 0,33 g du produit attendu après cristallisation dans le mélange DCM/éther iso, F = 159°C.
$\alpha_D^{25}$ = -226° (c = 0,1 ; chloroforme).

RMN $^1H$ : DMSO-$d_6$ : δ (ppm) : 2,0 : s : 3H ; 2,1 à 2,9 : m : 11H ; 3,6 à 4,0 : m : 11H ; 6,6 : mt : 3H ; 6,9 : t : 1H ; 7,1 : t : 1H ; 7, 9 à 8,0 : mt : 3H.

EXEMPLE 4

(3S)-4-[5-Chloro-1-[(2,4-diméthoxyphényl)sulfonyl]-3-(2-méthoxyphényl)-6-méthyl-2-oxo-2,3-dihydro-1*H*-indol-3-yl]-N,N-diméthylmorpholine-3-carboxamide, isomère lévogyre.

(I) : $R_1$ = Cl ; $R_2$ = 6-$CH_3$ ; $R_3$ = $OCH_3$ ; $R_4$ = H ; $R_5$ = -$N(CH_3)_2$ ; $R_6$ = 2-$OCH_3$ ; $R_7$ = $OCH_3$.

On refroidit à 0°C, sous atmosphère d'argon, un mélange de 0,875 g du composé obtenu à la Préparation 3.5 (isomère B) dans 8 ml de DMF, ajoute 0,09 g d'hydrure de sodium à 60 % dans l'huile et laisse sous agitation jusqu'à la fin du dégagement gazeux. On ajoute ensuite 0,49 g de chlorure de 2,4-diméthoxybenzènesulfonyle et laisse 3 heures sous agitation à TA. On verse le mélange réactionnel dans l'eau, extrait à l'AcOEt, lave la phase organique à l'eau, par une solution saturée de NaCl, sèche sur $Na_2SO_4$ et évapore le solvant sous vide. On chromatographie le résidu sur gel de silice en éluant par le mélange DCM/MeOH (97/3 ; v/v). On obtient 1,02 g du produit attendu après cristallisation dans le mélange DCM/éther iso, F = 215-219°C.
$\alpha_D^{25}$ = - 143,4° (c = 0,11; chloroforme).

**Revendications**

1. Composé de formule :

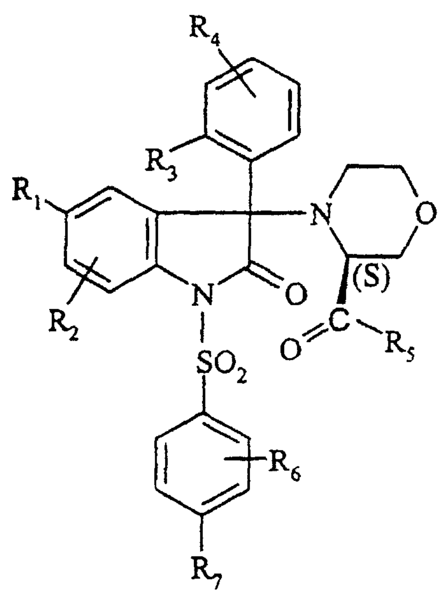

(I)

dans laquelle :

- $R_1$ représente un atome d'halogène ; un ($C_1$-$C_4$)alkyle ; un ($C_1$-$C_4$)alcoxy ; un radical trifluorométhyle ; un radical trifluorométhoxy ;
- R2 représente un atome d'hydrogène ; un atome d'halogène ; un ($C_1$-$C_4$)alkyle ; un ($C_1$-$C_4$)alcoxy ; un radical trifluorométhyle ;
- ou bien $R_2$ est en position -6- du cycle indol-2-one et $R_1$ et $R_2$ ensemble représentent le radical bivalent triméthylène ;
- $R_3$ représente un atome d'halogène ; un hydroxy ; un ($C_1$-$C_2$)alkyle ; un ($C_1$-$C_2$)alcoxy ; un radical trifluorométhoxy ;
- $R_4$ représente un atome d'hydrogène ; un atome d'halogène ; un ($C_1$-$C_2$)alkyle ; un ($C_1$-$C_2$)alcoxy ;
- ou bien $R_4$ est en position -3- du phényle et $R_3$ et $R_4$ ensemble représentent le radical méthylènedioxy ;
- $R_5$ représente un groupe éthylamino ; un groupe diméthylamino ; un radical azétidin-1-yle ; un ($C_1$-$C_2$)alcoxy ;
- $R_6$ représente un ($C_1$-$C_4$)alcoxy ;
- $R_7$ représente un ($C_1$-$C_4$)alcoxy ;

l'atome de carbone portant le substituant $COR_5$ a la configuration (S); et ses solvats et/ou hydrates.

**2.** Composé selon la revendication 1 sous forme d'isomères optiquement purs, l'atome de carbone en position 3 de l'indol-2-one a soit la configuration (R), soit la configuration (S).

**3.** Composé selon la revendication 2 sous forme d'isomère lévogyre.

**4.** Composé selon la revendication 1 à savoir le (3S)-4-[5-Chloro-1 -[(2,4-diméthoxyphényl)sulfonyl]-3-(2-méthoxyphényl)-2-oxo-2,3-dihydro-1*H*-indol-3-yl]-N,N-diméthylmorpholine-3-carboxamide, isomère lévogyre, ses solvats et/ou ses hydrates.

**5.** Composé selon la revendication 1 à savoir le (3S)-4-[6-Chloro-1-[(2,4-diméthoxyphényl)sulfonyl]-3-(2-méthoxyphényl)-5-méthyl-2-oxo-2,3-dihydro-1*H*-indol-3-yl]-N,N-diméthylmorpholine-3-carboxamide, isomère lévogyre, ses solvats et/ou ses hydrates.

**6.** Composé selon la revendication 1 à savoir le (3S)-4-[5-Chloro-1-[(2,4-diméthoxyphényl)sulfonyl]-3-(2-méthoxyphényl)-6-méthyl-2-oxo-2,3-dihydro-1*H*-indol-3-yl]-N,N-diméthylmorpholine-3-carboxamide, isomère lévogyre, ses solvats et/ou ses hydrates.

**7.** Procédé de préparation des composés de formule (I) selon la revendication 1, de leurs solvats et/ou hydrates **caractérisé en ce que** :

on fait réagir, en présence d'une base, un composé de formule :

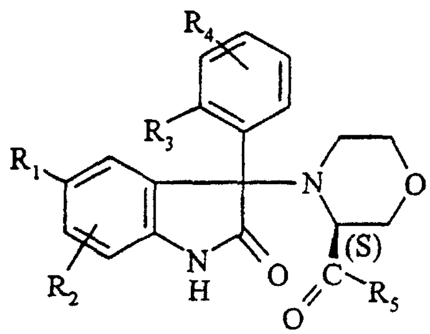

(II)

dans laquelle $R_1$, $R_2$, $R_3$, $R_4$ et $R_5$ sont tels que définis pour un composé de formule (I) dans la revendication 1, avec un halogénure de formule :

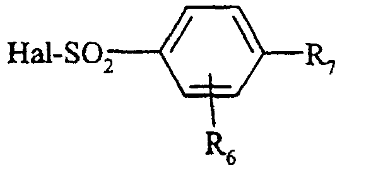

(III)

dans laquelle $R_6$ et $R_7$ sont tels que définis pour un composé de formule (I) dans la revendication 1 et Hal représente un atome d'halogène.

**8.** Composé de formule :

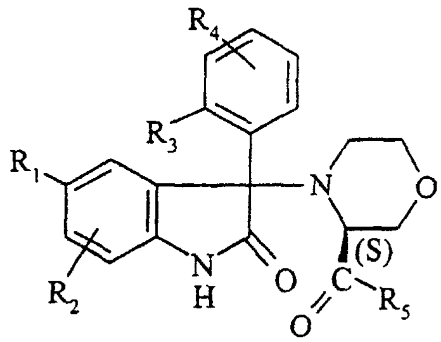

(II)

dans laquelle :

- $R_1$ représente un atome d'halogène ; un $(C_1-C_4)$alkyle ; un $(C_1-C_4)$alcoxy ; un radical trifluorométhyle ; un radical trifluorométhoxy ;
- $R_2$ représente un atome d'hydrogène ; un atome d'halogène ; un $(C_1-C_4)$alkyle ; un $(C_1-C_4)$alcoxy ; un radical trifluorométhyle ;
- ou bien $R_2$ est en position -6- du cycle indol-2-one et $R_1$ et $R_2$ ensemble représentent le radical bivalent triméthylène ;
- $R_3$ représente un atome d'halogène ; un hydroxy ; un $(C_1-C_2)$alkyle ; un $(C_1-C_2)$alcoxy ; un radical trifluorométhoxy ;
- $R_4$ représente un atome d'hydrogène ; un atome d'halogène ; un $(C_1-C_2)$alkyle ; un $(C_1-C_2)$alcoxy ;
- ou bien $R_4$ est en position -3- du phényle et $R_3$ et $R_4$ ensemble représentent le radical méthylènedioxy ;
- $R_5$ représente un groupe éthylamino ; un groupe diméthylamino ; un radical azétidin-1-yle ; un $(C_1-C_2)$alcoxy ;

et ses sels avec des acides minéraux ou organiques, sous forme d'isomère optiquement pur ou sous forme de mélange de diastéréoisomères.

**9.** Composition pharmaceutique comprenant en tant que principe actif un composé selon l'une quelconque des revendications 1 à 6, un de ses solvats et/ou hydrates pharmaceutiquement acceptable.

**10.** Utilisation d'un composé selon l'une quelconque des revendications 1 à 6, de ses solvats et/ou hydrates pharmaceutiquement acceptables, pour la préparation de médicaments destinés à traiter toute pathologie où l'arginine-vasopressine et/ou ses récepteurs $V_{1b}$ ou à la fois ses récepteurs $V_{1b}$ et ses récepteurs $V_{1a}$ sont impliqués.

**11.** Médicament **caractérisé en ce qu'**il est constitué d'un composé selon l'une quelconque des revendications 1 à 6.

## Patentansprüche

**1.** Verbindung der Formel:

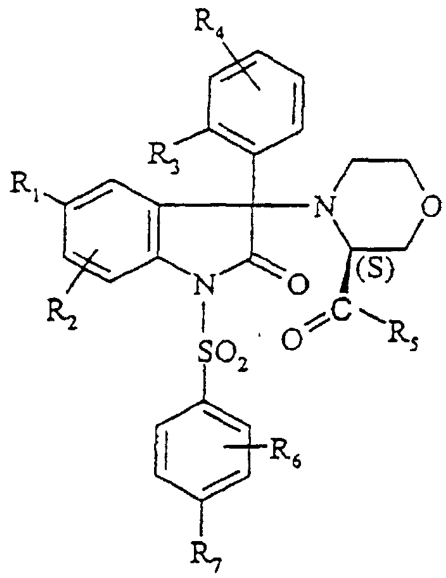

(I)

in der:

- $R_1$ ein Halogenatom; eine $(C_1-C_4)$-Alkylgruppe; eine $(C_1-C_4)$-Alkoxygruppe; eine Trifluormethylgruppe; oder eine Trifluormethoxygruppe bedeutet;
- $R_2$ ein Wasserstoffatom; ein Halogenatom; eine $(C_1-C_4)$-Alkylgruppe; eine $(C1-C_4)$-Alkoxygruppe; oder eine Trifluormethylgruppe darstellt;
- oder $R_2$ in der 6-Stellung des Indol-2-on-Ringes steht und $R_1$ und $R_2$ gemeinsam den zweiwertigen Trimethylen-Rest bilden;
- $R_3$ ein Halogenatom; eine Hydroxylgruppe; eine $(C_1-C_2)$-Alkylgruppe; eine $(C_1-C_2)$-Alkoxygruppe; oder eine Trifluormethoxygruppe bedeutet;
- $R_4$ ein Wasserstoffatom; ein Halogenatom; eine $(C_1-C_2)$-Alkylgruppe; oder eine $(C_1-C_2)$-Alkoxygruppe darstellt;
- oder $R_4$ in der 3-Stellung des Phenylrestes steht und $R_3$ und $R_4$ gemeinsam die Methylendioxygruppe bilden;
- $R_5$ eine Ethylaminogruppe; eine Dimethylaminogruppe; eine Azetidin-1-yl-gruppe oder eine $(C_1-C_2)$-Alkoxygruppe darstellt;
- $R_6$ eine $(C_1-C_4)$-Alkoxygruppe bedeutet;
- $R_7$ eine $(C_1-C_4)$-Alkoxygruppe darstellt;

wobei das den Substituenten $COR_5$ tragende Kohlenstoffatom in der Konfiguration (S) vorliegt;
und deren Solvate und/oder Hydrate.

**2.** Verbindung nach Anspruch 1 in Form der optisch reinen Isomeren. wobei das Kohlenstoffatom in der 3-Stellung des Indol-2-on-Ringes entweder in der Konfiguration (R) oder in der Konfiguration (S) vorliegt.

**3.** Verbindung nach Anspruch 2 in Form des linksdrehenden Isomeren.

**4.** Verbindung nach Anspruch 1, nämlich (3S)-4-[5-Chlor-1-[(2,4-dimethoxyphenyl)-sulfonyl]-3-(2-methoxyphenyl)-2-oxo-2,3-dihydro-1*H*-indol-3-yl]-N,N-dimethylmorpholin-3-carboxamid, linksdrehendes Isomeres, seine Solvate und/oder Hydrate.

**5.** Verbindung nach Anspruch 1, nämlich (3S)-4-[6-Chlor-1-[(2,4-dimethoxyphenyl)-sulfonyl]-3-(2-methoxyphenyl)-5-methyl-2-oxo-2,3-dihydro-1*H*-indol-3-yl]-N,N-dimethylmorpholin-3-carboxamid, linksdrehendes Isomeres, seine Solvate und/oder Hydrate.

**6.** Verbindung nach Anspruch 1, nämlich (3S)-4-[5-Chlor-1-[(2,4-dimethoxyphenyl)-sulfonyl]-3-(2-methoxyphenyl)-6-methyl-2-oxo-2,3-dihydro-1*H*-indol-3-yl]-N,N-dimethylmorpholin-3-carboxamid, linksdrehendes Isomeres, seine Solvate und/oder Hydrate.

**7.** Verfahren zur Herstellung der Verbindungen der Formel (I) nach Anspruch 1, von deren Solvaten und/oder Hydraten, **dadurch gekennzeichnet, daß** man:

eine Verbindung der Formel:

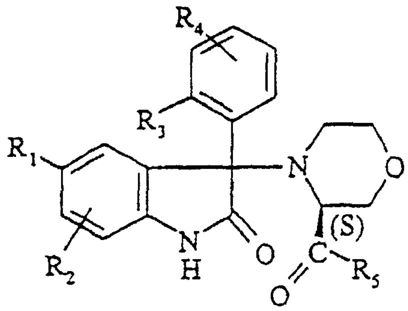

(II)

in der $R_1$, $R_2$, $R_3$, $R_4$ und $R_5$ die bezüglich der Formel (I) in Anspruch 1 angegebenen Bedeutungen besitzen, in Gegenwart einer Base mit einem Halogenid der Formel:

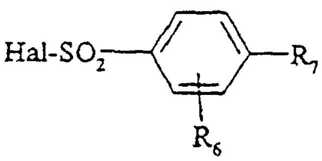

(III)

in der $R_6$ und $R_7$ die bezüglich einer Verbindung der Formel (I) in Anspruch 1 angegebenen Bedeutungen besitzen und Hal ein Halogenatom darstellt, umsetzt.

**8.** Verbindung der Formel:

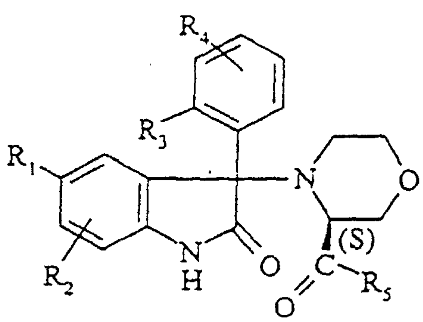

(II)

in der:

- $R_1$ ein Halogenatom; eine $(C_1\text{-}C_4)$-Alkylgruppe; eine $(C_1\text{-}C_4)$-Alkoxygruppe; eine Trifluormethylgruppe; oder eine Trifluormethoxygruppe bedeutet;
- $R_2$ ein Wasserstoffatom; ein Halogenatom; eine $(C_1\text{-}C_4)$-Alkylgruppe; eine $(C_1\text{-}C_4)$-Alkoxygruppe; oder eine Trifluormethylgruppe bedeutet;
- oder $R_2$ in der 6-Stellung des Indol-2-on-Ringes steht und $R_1$ und $R_2$ gemeinsam den zweiwertigen Trimethylen-Rest bilden;
- $R_3$ ein Halogenatom; eine Hydroxylgruppe; eine $(C_1\text{-}C_2)$-Alkylgruppe; eine $(C_1\text{-}C_2)$-Alkoxygruppe; oder eine Trifluormethoxygruppe bedeutet;
- $R_4$ ein Wasserstoffatom; ein Halogenatom; eine $(C_1\text{-}C_2)$-Alkylgruppe; oder eine $(C_1\text{-}C_2)$-Alkoxygruppe darstellt;
- oder $R_4$ in der 3-Stellung des Phenylrestes steht und $R_3$ und $R_4$ gemeinsam die Methylendioxygruppe bilden;

- $R_5$ eine Ethylaminogruppe; eine Dimethylaminogruppe; eine Azetidin-1-yl-gruppe oder eine ($C_1$-$C_2$)-Alkoxygruppe darstellt;

und deren Salze mit anorganischen oder organischen Säuren, in Form des optisch reinen Isomeren oder in Form einer Mischung der Diastereoisomeren.

**9.** Pharmazeutische Zubereitung. enthaltend als Wirkstoff eine Verbindung nach irgendeinem der Ansprüche 1 bis 6 oder eines ihrer pharmazeutisch annehmbaren Solvate und/oder Hydrate.

**10.** Verwendung einer Verbindung nach irgendeinem der Ansprüche 1 bis 6 und ihrer pharmazeutisch annehmbaren Solvate und/oder Hydrate für die Herstellung von Arzneimitteln zur Behandlung sämtlicher pathologischer Zustände, bei denen Arginin-Vasopressin und/oder seine Rezeptoren $V_{1b}$ oder gleichzeitig seine Rezeptoren $V_{1b}$ und seine Rezeptoren $V_{1a}$ beteiligt sind.

**11.** Arzneimittel, **dadurch gekennzeichnet, daß** es aus einer Verbindung nach irgendeinem der Ansprüche 1 bis 6 gebildet wird.

## Claims

**1.** Compound of formula:

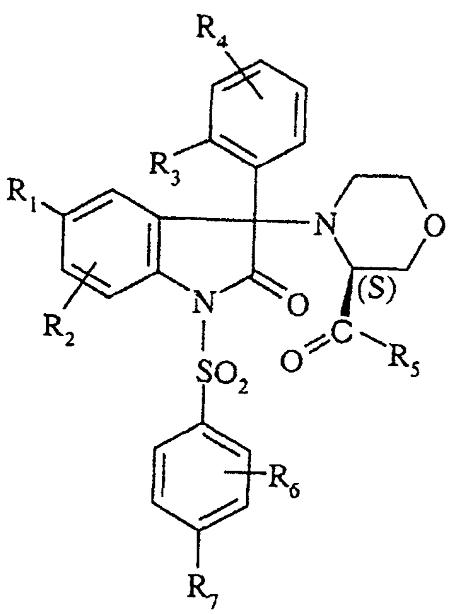

(I)

in which:

- $R_1$ represents a halogen atom; a ($C_1$-$C_4$)alkyl; a ($C_1$-$C_4$)alkoxy; a trifluoromethyl radical; or a trifluoromethoxy radical;
- $R_2$ represents a hydrogen atom; a halogen atom; a ($C_1$-$C_4$)alkyl; a ($C_1$-$C_4$)alkoxy; or a trifluoromethyl radical;
- or else $R_2$ is in the 6-position of the indol-2-one ring and $R_1$ and $R_2$ together represent the bivalent trimethylene radical;
- $R_3$ represents a halogen atom; a hydroxyl; a ($C_1$-$C_2$)alkyl; a ($C_1$-$C_2$)alkoxy; or a trifluoromethoxy radical;
- $R_4$ represents a hydrogen atom; a halogen atom; a ($C_1$-$C_2$)alkyl; or a ($C_1$-$C_2$)alkoxy;
- or else $R_4$ is in the 3-position of the phenyl and $R_3$ and $R_4$ together represent the methylenedioxy radical;
- $R_5$ represents an ethylamino group; a dimethylamino group; an azetidin-1-yl radical; or a ($C_1$-$C_2$)alkoxy;
- $R_6$ represents a ($C_1$-$C_4$)alkoxy;
- $R_7$ represents a ($C_1$-$C_4$)alkoxy;

the carbon atom bearing the $COR_5$ substituent is in the (S) configuration ; and its solvates and/or hydrates.

**2.** Compound according to Claim 1, in the form of optically pure isomers, the carbon atom in the 3-position of the indol-2-one has either the (R) configuration or the (S) configuration.

**3.** Compound according to Claim 2, in the form of a laevorotatory isomer.

**4.** Compound according to Claim 1, namely (3S)-4-[5-chloro-1-[(2,4-dimethoxyphenyl)sulphonyl]-3-(2-methoxyphenyl)-2-oxo-2,3-dihydro-1*H*-indol-3-yl]-N,N-dimethylmorpholine-3-carboxamide, laevorotatory isomer, its solvates and/or its hydrates.

**5.** Compound according to Claim 1, namely (3S)-4-[6-chloro-1-[(2,4-dimethoxyphenyl)sulphonyl]-3-(2-methoxyphenyl)-5-methyl-2-oxo-2,3-dihydro-1*H*-indol-3-yl]-N,N-dimethylmorpholine-3-carboxamide, laevorotatory isomer, its solvates and/or its hydrates.

**6.** Compound according to Claim 1, namely (3S)-4-[5-chloro-1-[(2,4-dimethoxyphenyl)sulphonyl]-3-(2-methoxyphenyl)-6-methyl-2-oxo-2,3-dihydro-1*H*-indol-3-yl]-N,N-dimethylmorpholine-3-carboxamide, laevorotatory isomer, its solvates and/or its hydrates.

**7.** Process for the preparation of the compounds of formula (I) according to Claim 1, their solvates and/or their hydrates, **characterized in that**:

a compound of formula:

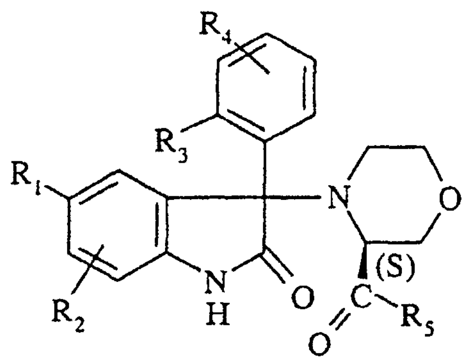

(II)

in which $R_1$, $R_2$, $R_3$, $R_4$ and $R_5$ are as defined for a compound of formula (I) in Claim 1, is reacted, in the presence of a base, with a halide of formula:

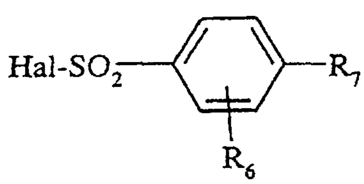

(III)

in which $R_6$ and $R_7$ are as defined for a compound of formula (I) in Claim 1 and Hal represents a halogen atom.

**8.** Compound of formula:

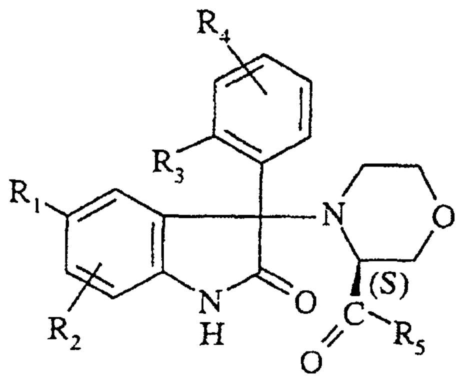

(II)

in which:

- $R_1$ represents a halogen atom; a $(C_1-C_4)$alkyl; a $(C_1-C_4)$alkoxy; a trifluoromethyl radical; or a trifluoromethoxy radical;
- $R_2$ represents a hydrogen atom; a halogen atom; a $(C_1-C_4)$alkyl; a $(C_1-C_4)$alkoxy; or a trifluoromethyl radical;
- or else $R_2$ is in the 6-position of the indol-2-one ring and $R_1$ and $R_2$ together represent the bivalent trimethylene radical;
- $R_3$ represents a halogen atom; a hydroxyl; a $(C_1-C_2)$alkyl; a $(C_1-C_2)$alkoxy; or a trifluoromethoxy radical;
- $R_4$ represents a hydrogen atom; a halogen atom; a $(C_1-C_2)$alkyl; or a $(C_1-C_2)$alkoxy;
- or else $R_4$ is in the 3-position of the phenyl and $R_3$ and $R_4$ together represent the methylenedioxy radical;
- $R_5$ represents an ethylamino group; a dimethylamino group; an azetidin-1-yl radical; or a $(C_1-C_2)$alkoxy;

and their salts with inorganic or organic acids, in the form of optically pure isomer or in the form of a mixture of diastereoisomers.

**9.** Pharmaceutical composition comprising, as active ingredient, a compound according to any one of Claims 1 to 6, one of its pharmaceutically acceptable solvates and/or hydrates.

**10.** Use of a compound according to any one of Claims 1 to 6, its pharmaceutically acceptable solvates and/or hydrates, for the preparation of medicaments intended for the treatment of any pathology where arginine-vasopressin and/or its $V_{1b}$ receptors or both its $V_{1b}$ receptors and its $V_{1a}$ receptors are implicated.

**11.** Medicament, **characterized in that** it consists of a compound according to any one of Claims 1 to 6.